# EUROPEAN PATENT APPLICATION

(11) **EP 3 800 468 A1**
(43) Date of publication of application: **07.04.2021**
(21) Application number: 20187839.4
(22) Date of filing: 27.07.2020
(51) Int. Cl.: G01N 23/04, G01N 23/18, G01N 33/12, G06T 7/00

(54) **INSPECTION DEVICE**

(30) Priority: 05.08.2019 JP 2019143735
(71) Applicant: Ishida Co., Ltd., Kyoto 606-8392 (JP)
(72) Inventor: KUDO, Daisuke, Ritto-shi, Shiga 520-3026 (JP); KOMORI, Haruhiko, Ritto-shi, Shiga 520-3026 (JP); YURUGI, Futoshi, Ritto-shi, Shiga 520-3026 (JP); IKEDA, Jun, Ritto-shi, Shiga 520-3026 (JP); YAMAMOTO, Yoichi, Ritto-shi, Shiga 520-3026 (JP); TODORI, Michihiko, Ritto-shi, Shiga 520-3026 (JP); SUGIMOTO, Kazuyuki, Ritto-shi, Shiga 520-3026 (JP)
(74) Representative: Global IP Europe Patentanwaltskanzlei

(57) **Abstract**

[Problem] It is an object of this invention to provide an inspection device that can present results of inspections of goods in a way by which one can easily distinguish between predetermined defects and other defects.

[Solution] An X-ray inspection device 10 inspects, on the basis of images of goods G, the goods G. The X-ray inspection device 10 includes a defect judging component 51c, a display component (a first display component 111 and a second display component 112), and an image adjusting component 51d. The defect judging component 51c judges, on the basis of the images of the goods G, whether or not the goods G have defects. The display component displays the images of the goods G. The defect judging component 51c judges whether the defects are first defects (contaminant C11) with a high need to be removed from the goods G or second defects (contaminant C12) other than the first defects. The image adjusting component 51d adjusts the images of the goods G that are displayed on the display component by configuring modes of display of the defects in the images of the goods G to differ between a case where the defects include the first defects and a case where the defects do not include the first defects.

## Description

### [Technical Field]

This invention relates to an inspection device for goods.

### [Background Art]

Conventionally, as disclosed in patent document JP-ANo. 2002-228761, an inspection device that irradiates goods such as foods with radiation such as X-rays to inspect the goods has been used. The inspection device inspects the goods by judging whether or not the goods have defects on the basis of images obtained by irradiating the goods with the radiation, for example. The defects are, for example, contaminants mixed in with the goods.

### [Summary of Invention]

### [Problem the Invention is to Solve]

In inspection devices that judge whether or not there are contaminants mixed in with goods, marking the positions of detected contaminants on images of the goods and displaying them on a screen, for example, has been widely employed. However, in a case where workers use the inspection device to remove the contaminants mixed in with the goods, it is difficult for the workers to distinguish at a glance whether the contaminants marked on the screen are contaminants that are alright for them not to remove or are contaminants that they should remove.

It is an object of this invention to provide an inspection device that can present results of inspections of goods in a way by which one can easily distinguish between predetermined defects and other defects.

### [Means for Solving the Problem]

An inspection device pertaining to a first aspect of the invention inspects, on the basis of images of goods, the goods. The inspection device includes a judging component, a display component, and an image adjusting component. The judging component judges, on the basis of the images of the goods, whether or not the goods have defects. The display component displays the images of the goods. The image adjusting component adjusts the images of the goods that are displayed on the display component. The judging component judges whether the defects are first defects with a high need to be removed from the goods or second defects other than the first defects. The image adjusting component adjusts the images of the goods by configuring modes of display of the defects in the images of the goods to differ between a case where the defects include the first defects and a case where the defects do not include the first defects.

This inspection device can present results of inspections of goods in a way by which one can easily distinguish between predetermined defects and other defects.

An inspection device pertaining to a second aspect of the invention is the inspection device pertaining to the first aspect, wherein the judging component judges that the goods have defects in a case where the judging component has detected at least one of contaminants included in the goods, abnormalities in the shape of the goods, and abnormalities in the number of the goods.

An inspection device pertaining to a third aspect of the invention is the inspection device pertaining to the first aspect or the second aspect, wherein the image adjusting component uses at least one of character strings, numerical values, figures, and frames surrounding predetermined regions of the images of the goods to adjust the images of the goods so that the mode of display of the first defects differs from the mode of display of the second defects.

An inspection device pertaining to a fourth aspect of the invention is the inspection device pertaining to any one of the first to third aspects, wherein the image adjusting component adjusts the images of the goods by configuring the modes of display of the defects in the images of the goods so that entireties of the goods are surrounded by frames in a case where the defects include the first defects.

This inspection device can present results of inspections of goods in a way by which one can easily distinguish between goods that have defects that should be removed and goods that do not have defects that should be removed.

An inspection device pertaining to a fifth aspect of the invention is the inspection device pertaining to any one of the first to fourth aspects, wherein the judging component judges that the goods have the first defects in a case where the goods have defects whose detection degree is higher than a predetermined threshold.

An inspection device pertaining to a sixth aspect of the invention is the inspection device pertaining to the fifth aspect, further including a setting component that sets the threshold.

An inspection device pertaining to a seventh aspect of the invention is the inspection device pertaining to the fifth aspect or the sixth aspect, wherein the image adjusting component adjusts the images of the goods so that information relating to the detection degree is included in the images of the goods.

An inspection device pertaining to an eighth aspect of the invention is the inspection device pertaining to any one of the fifth to seventh aspects, wherein the image adjusting component adjusts the images of the goods so that the mode of display of the first defects differs from the mode of display of the second defects in accordance with the difference between the detection degree and the threshold.

An inspection device pertaining to a ninth aspect of the invention is the inspection device pertaining to any one of the first to eighth aspects, wherein the image adjusting component adjusts the images of the goods so that, together with thumbnail images of the goods, enlarged images of the goods in the neighborhood of positions where there are defects are displayed on the display component.

This inspection device can present positions where there are defects that should be removed from the goods in a way that is easy to understand to workers who remove the defects from the goods.

An inspection device pertaining to a tenth aspect of the invention is the inspection device pertaining to any one of the first to ninth aspects, further comprising a sorting component that sorts to a predetermined region the goods that have been judged as having defects by the judging component.

### [Effects of Invention]

The inspection device pertaining to the invention can present results of inspections of goods in a way by which one can easily distinguish between predetermined defects and other defects.

### [Brief Description of Drawings]

FIG. 1 is a perspective view showing the outward appearance of an X-ray inspection device 10 that is an embodiment of the invention.
FIG. 2 is a schematic view of an inspection line 100 into which the X-ray inspection device 10 is incorporated.
FIG. 3 is a schematic view of the inside of a shield box 11 of the X-ray inspection device 10.
FIG. 4 is a drawing for describing the principle of X-ray inspections of goods G.
FIG. 5 is a block diagram of a control computer 50.
FIG. 6 is a drawing for describing the principle of X-ray inspections of goods G that have a contaminant C.
FIG. 7 is a drawing for describing the principle of X-ray inspections of goods G that have two types of contaminants C11, C12.
FIG. 8 is a drawing showing an example of an X-ray transmission image of a good G that has the two types of contaminants C11, C12.
FIG. 9 is a drawing showing the X-ray transmission image of FIG. 8 that has been adjusted.
FIG. 10 is a drawing showing the X-ray transmission image of FIG. 8 into which a character string F1 has been inserted in example modification B.
FIG. 11 is a drawing showing the X-ray transmission image of FIG. 8 into which numerical values F2 have been inserted in example modification B.
FIG. 12 is a drawing showing the X-ray transmission image of FIG. 8 into which a figure F3 has been inserted in example modification B.
FIG. 13 is an example of an image displayed on a first display component 111 and a second display component 112 in example modification E.
FIG. 14 is a drawing showing an X-ray transmission image that has been adjusted in example modification F.

### [Mode for Carrying out the Invention]

An embodiment of the invention will be described with reference to the drawings. The embodiment described below is a specific example of the invention and is not intended to limit the technical scope of the invention.

### (1) Overall Configuration of X-ray Inspection Device

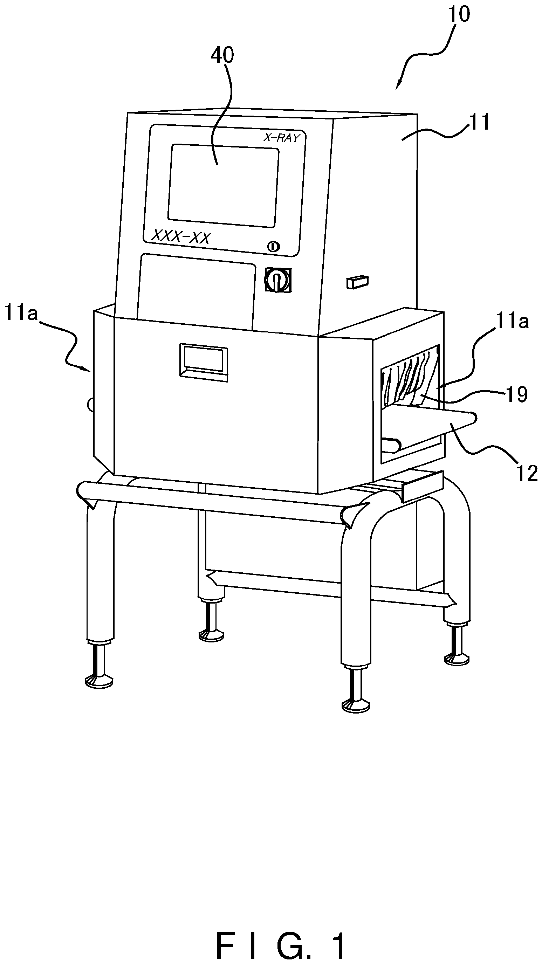

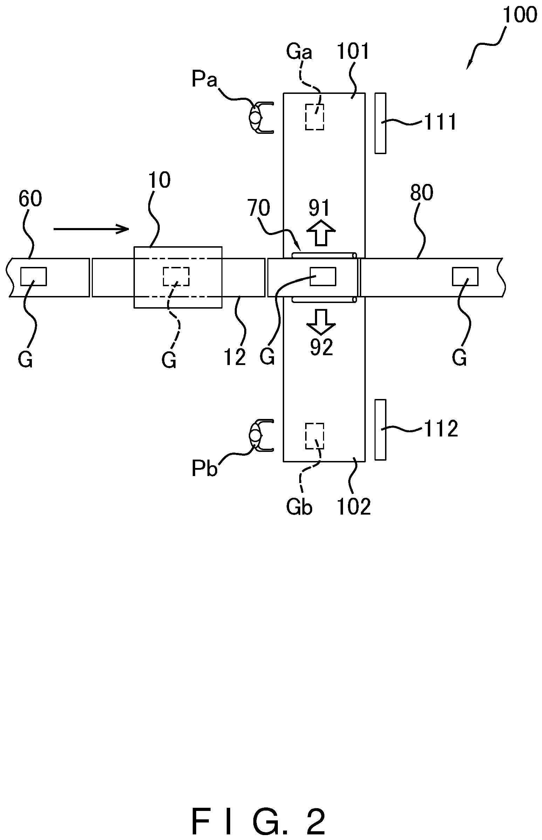

FIG. 1 is a perspective view showing the outward appearance of an X-ray inspection device 10 that is an embodiment of the inspection device pertaining to the invention. FIG. 2 is a schematic view of an inspection line 100 into which the X-ray inspection device 10 is incorporated. The inspection line 100 performs inspections of goods G. In the inspection line 100, the goods G are conveyed by an upstream conveyor 60 to the X-ray inspection device 10. In FIG. 2, the conveyance direction of the goods G is indicated by an arrow.

The X-ray inspection device 10 performs judgments about the non-defectiveness or defectiveness of the goods G by irradiating with X-rays the goods G that are continuously conveyed thereto by the upstream conveyor 60. Specifically, the X-ray inspection device 10 performs contamination inspections of the goods G and, on the basis of the inspection results, classifies the goods G into non-defective goods or defective goods. The results of the inspections by the X-ray inspection device 10 are sent to a sorting mechanism 70 disposed downstream of the X-ray inspection device 10. The sorting mechanism 70 sends the goods G that have been judged to be non-defective goods in the X-ray inspection device 10 to a downstream conveyor 80 that discharges the non-defective goods. The sorting mechanism 70 sorts, in defective good discharge directions 91, 92, the goods G that have been judged to be defective goods in the X-ray inspection device 10, thereby discharging them from the inspection line 100.

In this embodiment, the goods G are predetermined cuts of chicken meat. The chicken meat is obtained by cutting up raw chicken into its parts by machine or hand, and has predetermined dimensions or weights. The chicken meat carries the risk of including contaminants that should be removed by workers before shipment and contaminants that do not need to be removed. The contaminants that should be removed are, for example, bones and metal pieces. The contaminants that do not need to be removed are, for example, cartilage.

The X-ray inspection device 10 inspects the goods G by judging, on the basis of X-ray transmission images of the goods G, whether or not the goods G have defects. The X-ray inspection device 10 classifies the goods G that have defects into defective goods and classifies the goods G that do not have defects into non-defective goods. In this embodiment, "defects" are contaminants included in the goods G. The defects are divided into "first defects," which are contaminants that should be removed by workers, and "second defects," which are contaminants that do not need to be removed by workers. The second defects are defects other than the first defects. The X-ray inspection device 10 judges whether the defects included in the goods are the first defects or the second defects. The X-ray inspection device 10, when displaying the X-ray transmission images of the goods as the inspection results on a display for example, differentiates the mode of display of the first defects from the mode of display of the second defects.

The goods G that have been judged as having defects (contaminants) in the X-ray inspection device 10 and have been discharged from the inspection line 100 by the sorting mechanism 70 are sent to work lines 101, 102. On each of the work lines 101, 102, the contaminants included in goods Ga, Gb are checked by workers Pa, Pb. The workers Pa, Pb each remove the contaminants from the goods Ga, Gb as needed. The goods Ga, Gb from which the contaminants have been removed are sent to the upstream conveyor 60 and are inspected again by the X-ray inspection device 10.

### (2) Detailed Description of X-ray Inspection Device 10

The X-ray inspection device 10 is mainly configured from a shield box 11, a conveyance unit 12, an X-ray irradiator 20, a line sensor 30, a monitor 40, and a control computer 50.

### (2-1) Shield Box 11

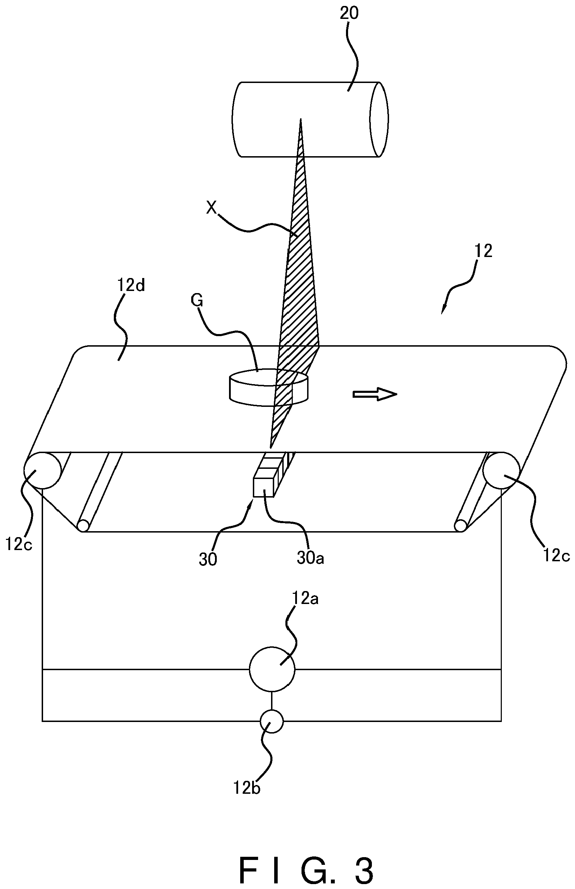

FIG. 3 is a schematic view of the inside of the shield box 11 of the X-ray inspection device 10. The shield box 11 is a casing of the X-ray inspection device 10. As shown in FIG. 1, in both side surfaces of the shield box 11 are formed openings 11a for conveying the goods G in and out. The openings 11a are used to convey the goods G from the outside of the shield box 11 to the inside or to convey the goods G from the inside of the shield box 11 to the outside. The openings 11a are blocked by shielding curtains 19. The shielding curtains 19 control leakage of X-rays from the inside of the shield box 11 to the outside. The shielding curtains 19 are formed of tungsten sheets. The shielding curtains 19 are pushed aside by the goods G when the goods G are conveyed in and out.

Inside the shield box 11 are contained the conveyance unit 12, the X-ray irradiator 20, the line sensor 30, and the control computer 50, for example. In the upper portion of the front face of the shield box 11 are disposed the monitor 40, input keys, and a power switch, for example.

### (2-2) Conveyance Unit 12

The conveyance unit 12 is a belt conveyor for conveying the goods G through the inside of the shield box 11. As shown in FIG. 1, the conveyance unit 12 is disposed so as to run through the openings 11a formed in both side surfaces of the shield box 11.

The conveyance unit 12 is mainly configured from a conveyor motor 12a, an encoder 12b, conveyor rollers 12c, and an endless belt 12d. The conveyor rollers 12c are driven by the conveyor motor 12a. The belt 12d is rotated, and the goods G on the belt 12d are conveyed, by the driving of the conveyor rollers 12c. In FIG. 3, the conveyance direction of the goods G is indicated by an arrow.

The speed at which the goods G are conveyed by the conveyance unit 12 changes in accordance with a set speed that has been input by the operator of the X-ray inspection device 10. The control computer 50 inverter-controls the conveyor motor 12a on the basis of the set speed to finely control the conveyance speed of the goods G. The encoder 12b of the conveyance unit 12 calculates the conveyance speed of the goods G by detecting the rotational speed of the conveyor motor 12a and sends it to the control computer 50.

Although the conveyance unit 12 uses a belt conveyor as the conveyance mechanism, instead of a belt conveyor it may also use a top chain conveyor and a rotating table, for example, as the conveyance mechanism.

### (2-3) X-ray Irradiator 20

The X-ray irradiator 20 is an X-ray source that irradiates with X-rays the goods G that have been conveyed by the conveyance unit 12 to a predetermined position inside the shield box 11. The X-rays emitted by the X-ray irradiator 20 include X-rays of various energies.

As shown in FIG. 3, the X-ray irradiator 20 is disposed above the conveyance unit 12. The X-ray irradiator 20 emits fan-shaped X-rays (radiation) toward the line sensor 30 disposed under the conveyance unit 12. An irradiation range X of the X-rays is, as shown in FIG. 3, perpendicular to a conveyance surface of the conveyance unit 12 and fans out in a direction orthogonal to the direction in which the goods G are conveyed by the conveyance unit 12. That is, the X-rays emitted by the X-ray irradiator 20 fan out in the width direction of the belt 12d.

### (2-4) Line Sensor 30

The line sensor 30 is a sensor that detects the X-rays emitted by the X-ray irradiator 20. Specifically, the line sensor 30 detects transmitted X-rays, which are X-rays that have passed through the goods G conveyed by the conveyance unit 12.

As shown in FIG. 3, the line sensor 30 is disposed under the belt 12d of the conveyance unit 12. The line sensor 30 is configured from plural X-ray detection elements 30a. The plural X-ray detection elements 30a are horizontally disposed in a straight line along a direction (the width direction of the belt 12d) orthogonal to the direction in which the goods G are conveyed by the conveyance unit 12.

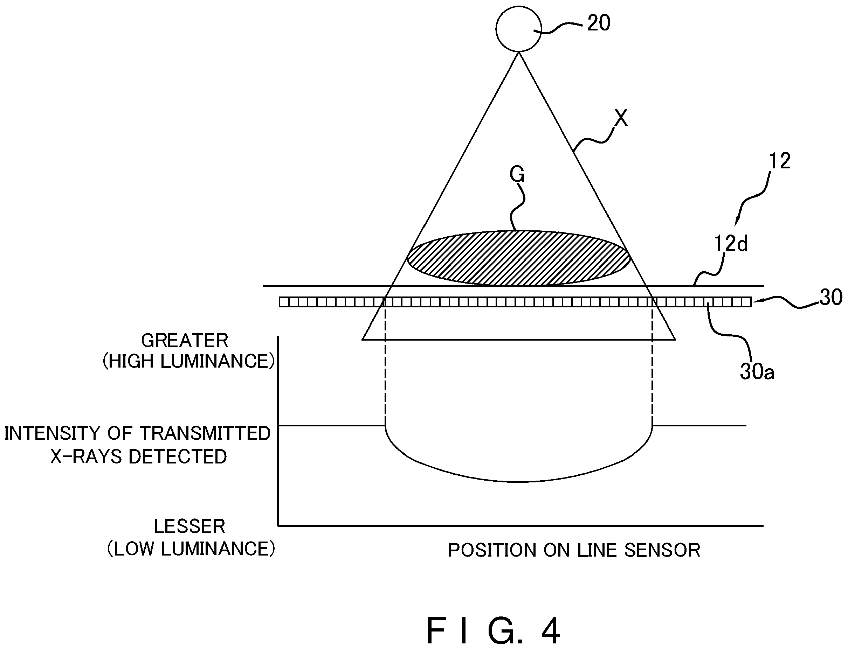

The line sensor 30 detects the transmitted X-rays and outputs X-ray transmission signals representing voltages according to the intensities of the transmitted X-rays that have been detected. The X-ray transmission signals are used to generate X-ray transmission images of the goods G as described later. FIG. 4 is a drawing for describing the principle of the X-ray inspections of the goods G. In FIG. 4, a graph of the intensities of the X-rays that have passed through a good G is shown. The horizontal axis of the graph represents the position on the line sensor 30. The vertical axis of the graph represents the intensity of the transmitted X-rays that the line sensor 30 has detected. In the X-ray transmission images of the goods G, places where the detected amount of transmitted X-rays is large appear brighter (have a higher luminance) and places where the detected amount of transmitted X-rays is small appear darker (have a lower luminance). That is, the contrast (luminance) in the X-ray transmission images of the goods G depends on the detected amount of transmitted X-rays. As shown in FIG. 4, the detected amount of X-rays that have passed through the goods G is lower than the detected amount of X-rays that have not passed through the goods G.

### (2-5) Monitor 40

The monitor 40 is, for example, a liquid crystal display with a touch panel function. The monitor 40 functions as an output component and an input component of the X-ray inspection device 10. The results of the inspections of the goods G, for example, are displayed on the monitor 40. Furthermore, screens for the input of initial settings and parameters relating to the judgments about the non-defectiveness or defectiveness of the goods G, for example, are displayed on the monitor 40.

The operator of the X-ray inspection device 10 can operate the monitor 40 to input inspection parameters and operation setting information, for example. The inspection parameters are parameters needed to determine the non-defectiveness or defectiveness of the goods G. Specifically, the inspection parameters are, for example, values of intensities of the transmitted X-rays used to determine whether or not there are contaminants included in the goods G. The operation setting information is information such as the inspection speed of the goods G and the conveyance direction of the conveyance unit 12.

The monitor 40 is connected to the control computer 50 and sends signals to, and receives signals from, the control computer 50. The inspection parameters and the operation setting information that have been input by the monitor 40 are stored in the control computer 50.

### (2-6) Control Computer 50

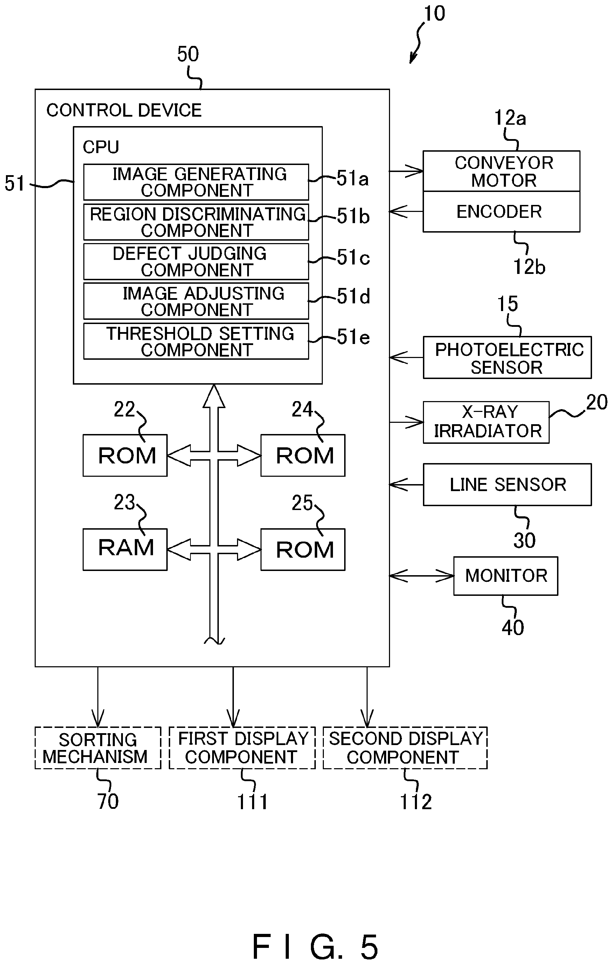

FIG. 5 is a block diagram of the control computer 50. The control computer 50 is mainly configured by a CPU 51, a ROM 52, a RAM 53, an HDD (hard disk drive) 54, and a media drive 55, for example. The elements 51 to 55 of the control computer 50 are connected to each other via bus lines such as an address bus and a data bus. Instead of the HDD 54, an SSD (solid state drive) may also be used. The media drive 55 is a device into which storage media such as CD-ROMs and USB memories, for example, are inserted and which is for reading data from, and writing data to, the storage media.

The CPU 51 executes various programs stored in the ROM 52 and the HDD 54. In the HDD 54 are saved and accumulated the inspection parameters and the inspection results, for example. The inspection parameters are capable of being set and changed by input from the operator using the touch panel function of the monitor 40. The operator can set these data so that they are saved and accumulated not only in the HDD 54 but also in the storage media inserted into the media drive 55.

The control computer 50 also includes a display control circuit, an input circuit, and communication ports that are not shown in the drawings. The display control circuit is a circuit that controls the display of the monitor 40. The input circuit is a circuit that imports input data that have been input by the operator via the touch panel of the monitor 40 and the input keys. The communication ports are ports that enable connection to external devices such as a printer and networks such as a LAN.

The control computer 50 is electrically connected to the conveyor motor 12a, the encoder 12b, a photoelectric sensor 15, the X-ray irradiator 20, the line sensor 30, and the monitor 40, for example. The photoelectric sensor 15 is a synchronous sensor for detecting the timings when the goods G pass through the irradiation range X of the fan-shaped X-rays (see FIG. 4). The photoelectric sensor 15 is mainly configured from a light emitter and a light receiver that form a pair disposed on either side of the conveyance unit 12. The control computer 50 acquires data relating to the rotational frequency of the conveyor motor 12a from the encoder 12b and, on the basis of the data, calculates the moving distances of the goods G.

Furthermore, the control computer 50 is electrically connected to the sorting mechanism 70 and a later-described first display component 111 and second display component 112.

### (3) Detailed Description of Control Computer 50

In the HDD 54 of the control computer 50 are stored various programs for executing the X-ray inspections of the goods G. The CPU 51 of the control computer 50, by reading and executing these programs, operates as an image generating component 51a, a region discriminating component 51b, a defect judging component 51c, an image adjusting component 51d, and a threshold setting component 51e.

### (3-1) Image Generating Component 51a

The image generating component 51a generates X-ray transmission images of the goods G on the basis of the X-ray transmission signals that are output from the line sensor 30. Specifically, the image generating component 51a acquires, in extremely small time intervals, the X-ray transmission signals that are output from each of the X-ray detection elements 30a of the line sensor 30 when the goods G pass through the irradiation range X of the fan-shaped X-rays (see FIG. 4) and, on the basis of the X-ray transmission signals it has acquired, generates the X-ray transmission images of the goods G.

The timings when the goods G pass through the irradiation range X of the fan-shaped X-rays are judged by signals from the photoelectric sensor 15. The image generating component 51a generates the X-ray transmission images of the goods G by joining together in a time series in a matrix the data per extremely small time interval relating to the brightness (luminance) of the X-rays obtained from each of the X-ray detection elements 30a of the line sensor 30. The X-ray transmission images that the image generating component 51a has generated are saved and accumulated in the ROM 52 and the HDD 54.

### (3-2) Region Discriminating Component 51b

The region discriminating component 51b discriminates the regions occupied by the goods G in the X-ray transmission images of the goods G that have been generated by the image generating component 51a. The region discriminating component 51b distinguishes between the regions occupied by the goods G and other regions on the basis of the luminance of each of the pixels in the X-ray transmission images of the goods G, for example. The discrimination results of the region discriminating component 51b are sent to the defect judging component 51c.

### (3-3) Defect Judging Component 51c

The defect judging component 51c detects defects (contaminants) included in the goods G by binarizing the X-ray transmission images of the goods G that have been generated by the image generating component 51a. More specifically, in a case where there is a region that appears darker than a preset value in the regions occupied by the goods G in the X-ray transmission images of the goods G, the defect judging component 51c judges that there is a contaminant mixed in with those goods G. The regions occupied by the goods G are the regions discriminated by the region discriminating component 51b.

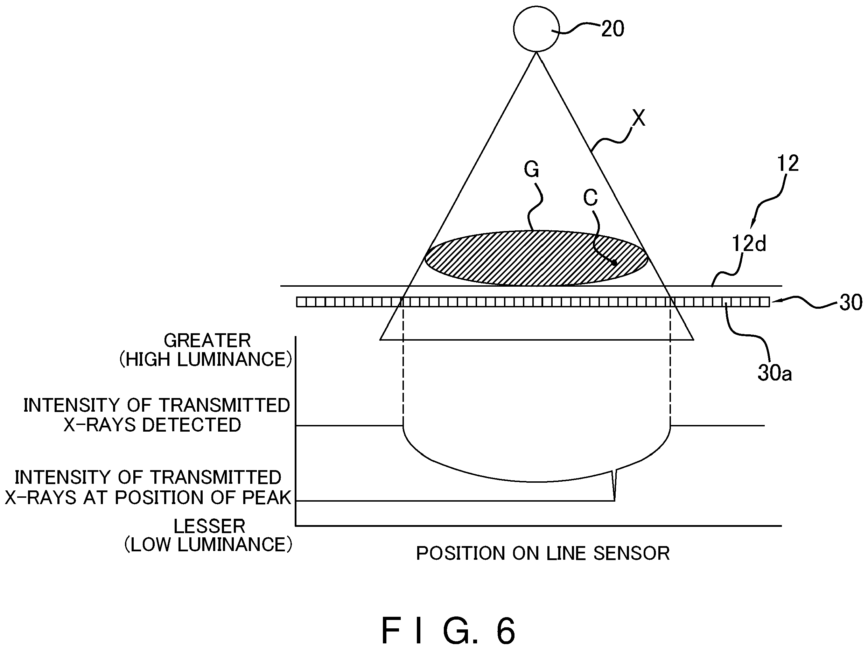

FIG. 6 is the same drawing as FIG. 4 and is for describing the principle of the X-ray inspections of the goods G that have a contaminant C. In FIG. 6, a graph of the intensities of the X-rays that have passed through a good G that has a contaminant C is shown. The contaminants C normally are difficult for the X-rays to pass through compared to the goods G. For that reason, as shown in FIG. 6, the detected amount of X-rays that have passed through the contaminant C in the good G is lower than the detected amount of X-rays that have passed through the good G but have not passed through the contaminant C. In FIG. 6, a peak at which the detected amount of X-rays is lower than that of the surrounding area is shown in the position of the contaminant C. In a case where the detected amount at the position of this peak is lower than a predetermined value, the defect judging component 51c judges that there is a contaminant C in the position of this peak.

Furthermore, the defect judging component 51c, in a case where it has detected defects (contaminants) included in the goods G, judges whether the defects are the first defects or the second defects. Specifically, in a case where the goods G have defects in which the detection degree of the contaminants C is higher than a predetermined value, the defect judging component 51c judges that the goods G have the first defects.

Here, the "detection degree" of the contaminants C will be described. As described above, the first defects are defects (contaminants) with a high need to be removed from the goods G, and the second defects are defects (contaminants) other than the first defects. In a case where the goods G are chicken meat, the detected amount of X-rays that pass through contaminants C that are the first defects (e.g., bones and metal pieces) tends to be lower than the detected amount of X-rays that pass through contaminants C that are the second defects (e.g., cartilage). For that reason, the lower the detected amount of X-rays that pass through the contaminants C is, the higher the probability is that the contaminants C are the first defects with a high need to be removed from the goods G. In this case, it is preferred that the detection degree of the contaminants C be a parameter where the detection degree becomes higher the lower the detected amount of X-rays that pass through the contaminants C is. For example, the detection degree of the contaminants C is the gray level of the pixels in the regions occupied by the contaminants C in the X-ray transmission images of the goods G. The gray level of the pixels in the X-ray transmission images corresponds to the detected amount of X-rays. Specifically, the higher the gray level of the pixels is, the lower the detected amount of X-rays in those pixels is. For that reason, the gray level of the pixels is a parameter that can be used as the detection degree of the contaminants C. The defect judging component 51c judges that defects (contaminants) whose detection degree found from the detected amount of X-rays that have passed through the goods G is higher than a predetermined detection threshold are the first defects.

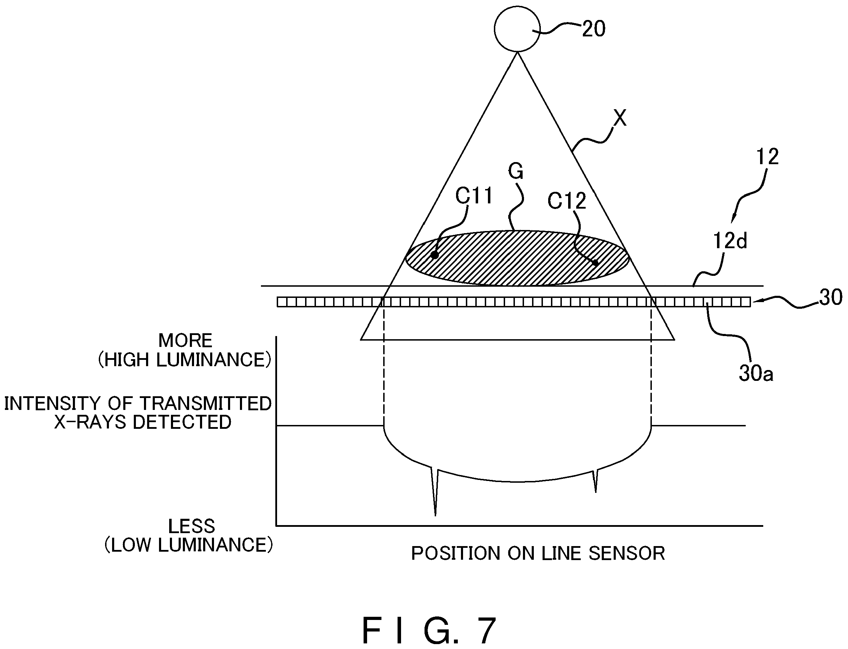

FIG. 7 is the same drawing as FIG. 4 and is for describing the principle of the X-ray inspections of the goods G that have two types of contaminants C11, C12. In FIG. 7, a graph of the intensities of the X-rays that have passed through a good G that has the two types of contaminants C11, C12 is shown. The contaminant C11 is a first defect. The contaminant C12 is a second defect. As shown in FIG. 7, the detected amounts of X-rays that have passed through the contaminants C11, C12 are lower than the detected amount of X-rays that have passed through the good G but have not passed through the contaminants C11, C12. Furthermore, the detected amount of X-rays that have passed through the contaminant C11 is lower than the detected amount of X-rays that have passed through the contaminant C12. For that reason, the gray level of the pixels in the region occupied by the contaminant C11 in the X-ray transmission image of the good G is higher than the gray level of the pixels in the region occupied by the contaminant C12. That is, the detection degree of the contaminant C11 is higher than the detection degree of the contaminant C12. In a case where the detection degree of the contaminant C11 is higher than the predetermined detection threshold and the detection degree of the contaminant C12 is the same as or lower than the predetermined detection threshold, the defect judging component 51c judges that the contaminant C11 is a first defect and that the contaminant C12 is a second defect. That said, however, the detection degree of the contaminant C12 needs to be higher than a predetermined value that is smaller than the predetermined detection threshold.

In this way, the defect judging component 51c can judge whether the defects included in the goods G are the first defects or the second defects by comparing the detection degree of the contaminants obtained on the basis of the detected amount of X-rays and the predetermined detection threshold. For that reason, the defect judging component 51c can discriminate regions occupied by the first defects (contaminant C11) and regions occupied by the second defects (contaminant C12) in the X-ray transmission images of the goods G. The defect judging component 51c discriminates regions occupied by the first defects and regions occupied by the second defects on the basis of the gray levels of each of the pixels in the X-ray transmission images of the goods G as the detection degrees, for example. Regions where the gray level of the pixels is high means regions where the detection degree of the contaminants C11, C12 is high. The judgment results of the defect judging component 51c are sent to the image adjusting component 51d.

### (3-4) Image Adjusting Component 51d

The image adjusting component 51d adjusts the X-ray transmission images of the goods G. Specifically, the image adjusting component 51d changes, on the basis of the judgment results of the defect judging component 51c, the X-ray transmission images of the goods G that have been generated by the image generating component 51a. The X-ray transmission images of the goods G that have been adjusted by the image adjusting component 51d are saved and accumulated in the ROM 52 and the HDD 54 and are displayed on the later-described first display component 111 and second display component 112.

The image adjusting component 51d adjusts the X-ray transmission images of the goods G so that the mode of display of the first defects differs from the mode of display of the second defects in a case where the defect judging component 51c has judged that the goods G have the first defects.

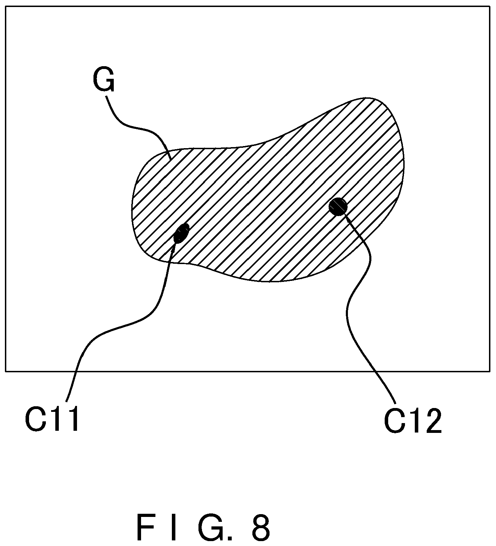

FIG. 8 is a drawing showing an example of an X-ray transmission image of a good G that has the two types of contaminants C11, C12. FIG. 8 is an image before being adjusted by the image adjusting component 51d. The good G is a specific cut of chicken meat. The good G has the two types of contaminants C11, C12. The contaminant C11 is a "first defect" that is a contaminant that should be removed by workers. The contaminant C12 is a "second defect" that is a contaminant that does not need to be removed by workers. The defect judging component 51c judges that the contaminant C11 is a first defect and judges that the contaminant C12 is a second defect, and the image adjusting component 51d receives the judgment results of the defect judging component 51c.

The X-ray transmission image shown in FIG. 8 is configured from plural pixels. Each of the pixels in the X-ray transmission image has one of multiple gray levels. In FIG. 8, the gray levels of the X-ray transmission image are indicated by the spacing between the hatching lines. Specifically, the narrower the spacing between the hatching lines of a certain region is, the higher the gray level of the pixels configuring that region is. In FIG. 8, the gray levels of the pixels of the contaminants C11, C12 are higher than the gray levels of the pixels of the good G outside the contaminants C11, C12.

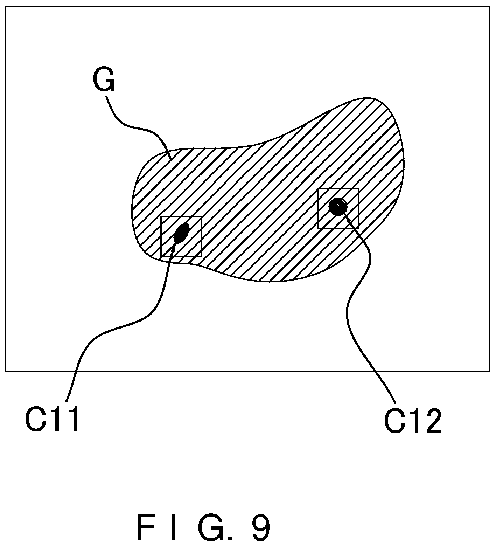

FIG. 9 is the X-ray transmission image of the good G shown in FIG. 8 after being adjusted by the image adjusting component 51d. The image adjusting component 51d acquires, on the basis of the judgment results of the defect judging component 51c, the region occupied by the first defect (contaminant C11) and the region occupied by the second defect (contaminant C12) in the X-ray transmission image of the good G. The image adjusting component 51d adjusts the X-ray transmission image of the good G so that, of the contaminants C11, C12, the mode of display of the contaminant C11 that is a first defect differs from the mode of display of the contaminant C12 that is a second defect. Specifically, the image adjusting component 51d uses at least one of character strings, numerical values, figures, and frames surrounding predetermined regions of the image of the good G to adjust the X-ray transmission image of the good G so that the mode of display of the first defect differs from the mode of display of the second defect.

In FIG. 9, the regions occupied by each of the contaminants C11, C12 are surrounded by rectangular frames so that workers easily see them. However, the frame surrounding the region occupied by the contaminant C11 that is a first defect is thicker than the frame surrounding the region occupied by the contaminant C12 that is a second defect, whereby the mode of display of the first defect differs from the mode of display of the second defect. Because of this, the first defect is more conspicuous to workers than the second defect.

### (3-5) Threshold Setting Component 51e

The threshold setting component 51e sets the detection threshold that was described in the section "(3-3) Defect Judging Component 51c." Specifically, the threshold setting component 51e displays on the monitor 40 a screen for setting the detection threshold and has the operator of the X-ray inspection device 10 input the detection threshold.

### (4) Operation of Identifying Positions of Contaminants

As shown in FIG. 2, the goods G that have been judged as having defects by the X-ray inspection device 10 are discharged from the inspection line 100 by the sorting mechanism 70 and sent to the work lines 101, 102. On the work lines 101, 102, the work of removing the contaminants is performed by the workers Pa, Pb. Here, the contaminants are the first defects that should be removed from the goods G. The second defects do not necessarily need to be removed on the work lines 101, 102. For that reason, in the work of removing the contaminants that are the first defects, it is necessary to identify beforehand the positions of the first defects mixed in with the goods G and present them to the workers Pa, Pb in a way that is easy to understand.

FIG. 8 is an X-ray transmission image of a good G before being adjusted by the image adjusting component 51d. In FIG. 8, the gray levels of the pixels in the regions occupied by the two contaminants C11, C12 are displayed higher than the gray levels of the pixels in the other region. The region occupied by the contaminant C11 represents a first defect, and the region occupied by the contaminant C12 represents a second defect. However, in a case where the gray levels of the pixels in the region occupied by the contaminant C11 are about the same as the gray levels of the pixels in the region occupied by the contaminant C12, it is difficult for the workers Pa, Pb to discriminate at a glance the region occupied by the contaminant C11 (first defect) even if they look at the X-ray transmission image shown in FIG. 8.

FIG. 9 is the X-ray transmission image of the good G after being adjusted by the image adjusting component 51d. In FIG. 9, of the two contaminants C11, C12, the mode of display of the contaminant C11 that is a first defect differs from the mode of display of the contaminant C12 that is a second defect. Specifically, the frame surrounding the region occupied by the contaminant C11 that is a first defect is thicker than the frame surrounding the region occupied by the contaminant C12 that is a second defect. Because of this difference in modes of display, the workers Pa, Pb can discriminate at a glance the region occupied by the contaminant C11 (first defect) by looking at the X-ray transmission image shown in FIG. 9. That is, the workers Pa, Pb can easily grasp the position of the first defect mixed in with the good G. Below, the X-ray transmission image of the good G shown in FIG. 9 that has been adjusted by the image adjusting component 51d will be called a contaminant position identifying image.

### (5) Process of Removing Contaminants

The process by which the workers Pa, Pb remove the contaminants included in the goods Ga, Gb on the work lines 101, 102 as shown in FIG. 2 will be described.

The goods Ga, Gb that have been judged as having defects (contaminants) in the X-ray inspection device 10 are sorted in the defective good discharge directions 91, 92 by the sorting mechanism 70 and discharged from the inspection line 100. The goods Ga, Gb that have been discharged are sent to the work lines 101, 102. On each of the work lines 101, 102, the contaminants included in the goods Ga, Gb are checked by the workers Pa, Pb. The work lines 101, 102 comprise a first work line 101 and a second work line 102.

The first work line 101 is disposed on the defective good discharge direction 91 side, and the second work line 102 is disposed on the defective good discharge direction 92 side. A first display component 111 is installed on the first work line 101, and a second display component 112 is installed on the second work line 102. The first display component 111 and the second display component 112 are monitors such as liquid crystal displays and are separate from the monitor 40 of the X-ray inspection device 10.

On the first work line 101, the worker Pa performs the work of removing the contaminants from the goods Ga in a position facing the first display component 111. On the second work line 102, the worker Pb performs the work of removing the contaminants from the goods Gb in a position facing the second display component 112.

As the goods Ga judged as having defects are discharged in the defective good discharge direction 91 and contaminant removal work is performed on the work line 101, the other goods Gb judged as having defects are discharged in the defective good discharge direction 92 and contaminant removal work is performed on the second work line 102.

The control computer 50 of the X-ray inspection device 10 sends to the first display component 111 the contaminant position identifying images of the goods Ga that have been sorted to the first work line 101 and sends to the second display component 112 the contaminant position identifying images of the goods Gb that have been sorted to the second work line 102.

On the first work line 101, the contaminant position identifying images of the goods Ga that are sent from the X-ray transmission device 10 are displayed on the first display component 111. The worker Pa checks the positions of the first defects (contaminants) included in the goods Ga and removes the first defects while looking at the contaminant position identifying images displayed on the first display component 111.

On the second work line 102, the contaminant position identifying images of the goods Gb that are sent from the X-ray transmission device 10 are displayed on the second display component 112. The worker Pb checks the positions of the first defects (contaminants) included in the goods Gb and removes the first defects while looking at the contaminant position identifying images displayed on the second display component 112.

### (6) Characteristics

In conventional X-ray inspection devices, a method in which the positions of detected contaminants are marked in X-ray transmission images of goods to make it easier for workers who will remove the contaminants to grasp the positions of the contaminants has been widely employed. However, in a case where workers remove by hand contaminants remaining inside chicken meat that is a good, it is difficult for the workers to distinguish at a glance between cartilage that is normally alright for them not to remove and bones or metal pieces that they should remove. Furthermore, the shapes of the contaminants are not consistent, and it is difficult to detect with high precision only the contaminants that they should remove. For that reason, in order to improve detection precision, they must tolerate misdetection to a certain extent. Furthermore, workers must remove only specific contaminants while looking at the output screens of the X-ray transmission images of the goods, so work efficiency tends to drop.

In the X-ray inspection device 10 of the embodiment, as shown in the contaminant position identifying image of the good G in FIG. 9, the regions occupied by the contaminants C11, C12 in the good G are surrounded by frames. Furthermore, the frame surrounding the region occupied by the contaminant C11 that is a first defect that should be removed is thicker than the frame surrounding the region occupied by the contaminant C12 that is a second defect that does not need to be removed. For that reason, the region representing the first defect in the contaminant position identifying image of the good G is displayed in a way that is more conspicuous to workers than the region representing the second defect. Because of this, workers can easily grasp the positions of the contaminants (defects) and can easily distinguish between the first defect and the second defect by looking at the output screens of the contaminant position identifying image of the good G. Consequently, workers can distinguish at a glance whether the contaminants included in the goods G (chicken meat) are the first defects (e.g., bones and metal pieces) that they should remove or are the second defects (e.g., cartilage) that are alright for them not to remove. As a result, the efficiency of the work of removing the contaminants from the goods G by the workers is improved, and the precision of contaminant removal is also improved.

### (7) Example Modifications

An embodiment of the invention has been described above, but the invention is not limited to the above embodiment, and various changes can be made thereto without departing from the scope of the invention.

### (7-1) Example Modification A

In the embodiment, the defect judging component 51c judges that the goods G have defects in a case where contaminants included in the goods G have been detected. However, the defects are not limited to contaminants included in the goods G. For example, the defect judging component 51c may also judge that the goods G have defects in a case where at least one of contaminants included the goods G, abnormalities in the shape of the goods G, and abnormalities in the number of the goods G has been detected.

In a case where the defects in the goods G are abnormalities in the shape of the goods G, the defect judging component 51c may judge that the goods G have defects in a case where the difference between the shape of the goods G that are inspection targets and a shape serving as a standard is equal to or greater than a predetermined amount.

In a case where the defects in the goods G are abnormalities in the number of the goods G, the defect judging component 51c may judge that the goods G have defects in a case where the difference between the number of the goods G that are inspection targets and a number serving as a standard is equal to or greater than a predetermined amount. In this case, the goods G are, for example, goods in which plural products are packaged.

Furthermore, in the embodiment, the goods G are predetermined cuts of chicken meat. However, the goods G are not limited to chicken meat and may be any arbitrary product that needs to meet a predetermined quality standard.

### (7-2) Example Modification B

In the embodiment, the image adjusting component 51d adjusts the X-ray transmission images of the goods G so that, as shown in FIG. 9, the frame surrounding the region occupied by the contaminant C11 that is a first defect is thicker than the frame surrounding the region occupied by the contaminant C12 that is a second defect. Because of this, the image adjusting component 51d differentiates the mode of display of the contaminant C11 that is a first defect from the mode of display of the contaminant C12 that is a second defect. Because of this difference in modes of display, the workers Pa, Pb can discriminate at a glance the region occupied by the contaminant C11 (first defect) by looking at the X-ray transmission image shown in FIG. 9.

However, the image adjusting component 51d may also differentiate the mode of display of the first defects from the mode of display of the second defects by another method. For example, the image adjusting component 51d may use at least one of character strings, numerical values, figures, and frames surrounding predetermined regions of the images of the goods G to differentiate the mode of display of the first defects from the mode of display of the second defects. Next, cases where the image adjusting component 51d uses each of character strings, numerical values, and figures will be described.

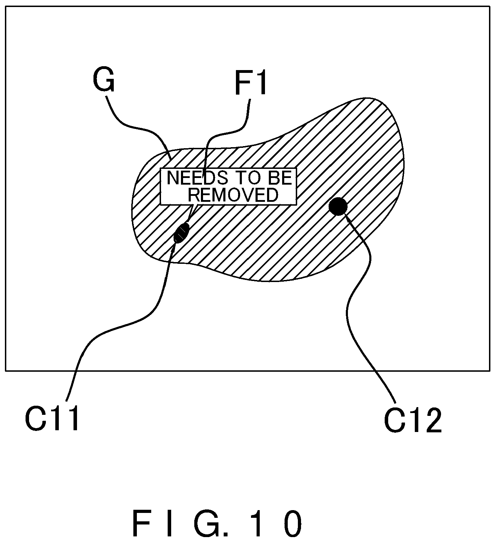

In the case of using character strings, the image adjusting component 51d inserts a predetermined character string into the X-ray transmission images so that workers can discriminate at a glance between the first defects and the second defects. FIG. 10 is a drawing showing the X-ray transmission image of FIG. 8 into which a character string F1 has been inserted. The character string F1 is disposed in the neighborhood of the region representing the contaminant C11 that is a first defect. In FIG. 10, the character string F1 is "Needs to be Removed," indicating that the contaminant C11 in the neighborhood of the display position of the character string F1 is a first defect that should be removed. Because of this, workers can easily grasp the position of the first defect. The character string F1 may be any arbitrary character string by which workers can easily grasp the first defects and the second defects.

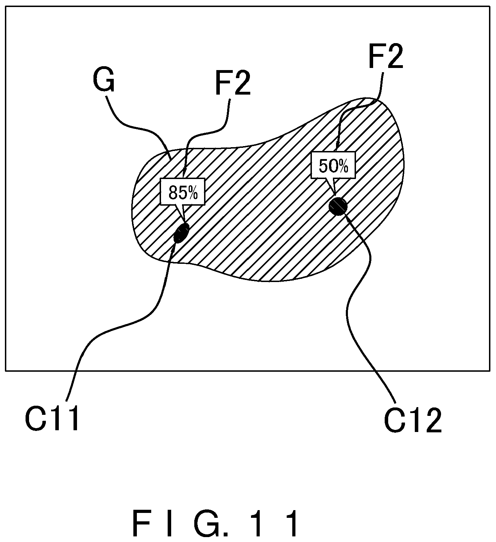

In the case of using numerical values, the image adjusting component 51d inserts a predetermined numerical value into the X-ray transmission images so that workers can discriminate at a glance between the first defects and the second defects. FIG. 11 is a drawing showing the X-ray transmission image of FIG. 8 into which numerical values F2 have been inserted. The numerical values F2 are disposed in the neighborhoods of the regions representing each of the contaminant C11 and the contaminant C12. In FIG. 11, the numerical values F2 are numerical values representing the "detection degree" described in the section "(3-3) Defect Judging Component 51c." The numerical values F2 represent the detection degrees of the contaminants C11, C12 in the neighborhoods of the display positions of the numerical values F2. The detection degrees are values ranging from 0% to 100%. For example, a contaminant whose detection degree is higher than 70% is a first defect, and a contaminant whose detection degree is equal to or less than 70% is a second defect. In this case, workers can easily distinguish between the first defects and the second defects by looking at the numerical values F2. The numerical values F2 may be any arbitrary parameter by which workers can easily distinguish between the first defects and the second defects.

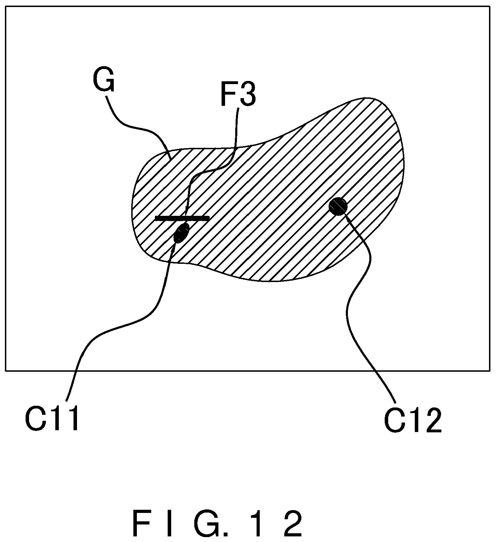

In the case of using figures, the image adjusting component 51d inserts a predetermined figure into the X-ray transmission images so that workers can discriminate at a glance between the first defects and the second defects. FIG. 12 is a drawing showing the X-ray transmission image of FIG. 8 into which a figure F3 has been inserted. The figure F3 is a bar-shaped figure disposed in the neighborhood of the region representing the contaminant C11 that is a first defect. In FIG. 12, the figure F3 indicates that the contaminant C11 in the neighborhood of the display position of the figure F3 is a first defect that should be removed. Because of this, workers can easily grasp the position of the first defect. The figure F3 may be any arbitrary figure by which workers can easily grasp the first defects and the second defects.

In the embodiment and this example modification, the image adjusting component 51d may also use mutually different colors to differentiate the mode of display of the first defects from the mode of display of the second defects. For example, in FIG. 9, the image adjusting component 51d may adjust the X-ray transmission image of the good G so that the frame surrounding the region occupied by the contaminant C11 that is a first defect is displayed in red and the frame surrounding the region occupied by the contaminant C12 that is a second defect is displayed in black.

### (7-3) Example Modification C

In example modification B, as shown in FIG. 11, the image adjusting component S1d may also insert into the X-ray transmission images of the goods G the numerical values F2 representing the detection degrees of the contaminants C11, C12. However, the image adjusting component 51d may also insert into the X-ray transmission images of the goods G information relating to the detection degrees in another format. For example, the image adjusting component 51d may insert into the X-ray transmission images of the goods G a list of the detection degrees of each of the contaminants that have been detected. In that case, the image adjusting component 51d may adjust the X-ray transmission images of the goods G so that workers easily see the positions of the contaminants corresponding to each of the detection degrees.

### (7-4) Example Modification D

The image adjusting component 51d may also adjust the X-ray transmission images of the goods G so that the mode of display of the first defects differs from the mode of display of the second defects in accordance with the difference between the detection degrees of contaminants that have been detected and the predetermined detection threshold. For example, in the case of using frames surrounding predetermined regions of the images of the goods G as in the embodiment, the image adjusting component 51d may adjust the X-ray transmission images of the goods G so that the larger a value obtained by subtracting the detection threshold from the detection degree is, the thicker the frame becomes.

### (7-5) Example Modification E

The image adjusting component 51d may also adjust the X-ray transmission images of the goods G so that, together with thumbnail images of the goods G, enlarged images of the goods G in the neighborhood of positions where there are defects are displayed on the first display component 111 and the second display component 112.

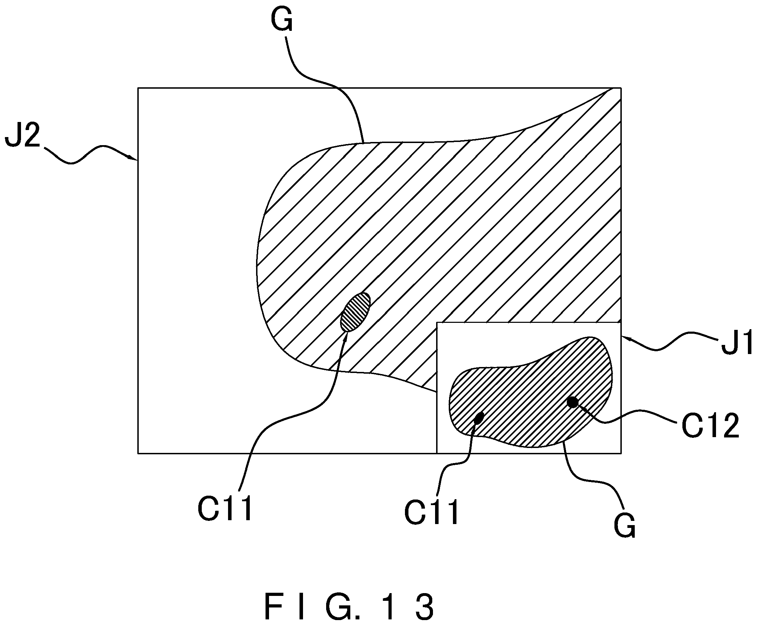

FIG. 13 is an example of an image that has been adjusted by the image adjusting component 51d in this example modification and is displayed on the first display component 111 and the second display component 112. This image includes a thumbnail image J1 of the good G and an enlarged image J2 of the good G. The thumbnail image J1 is, as shown in FIG. 8 of the embodiment, an image showing the entirety of the good G. The enlarged image J2 is, for example, an image of the good G in the neighborhood of the position of the first defect. The enlarged image J2 is an image in which part of the good G displayed in the thumbnail image J1 is enlarged. The enlarged image J2 is displayed on the entire screens of the first display component 111 and the second display component 112, and the thumbnail image J1 is displayed overlying the enlarged image J2 on parts of the screens of the first display component 111 and the second display component 112.

In this example modification, workers can easily grasp the position and shape of a contaminant that is a first defect by looking at the enlarged image J2, and can easily identify contaminants in the contaminant removal work.

In this example modification, the image adjusting component 51d may also adjust at least one of the thumbnail image J1 and the enlarged image J2 so that the mode of display of the first defects differs from the mode of display of the second defects as shown in FIGS. 9 to 12.

### (7-6) Example Modification F

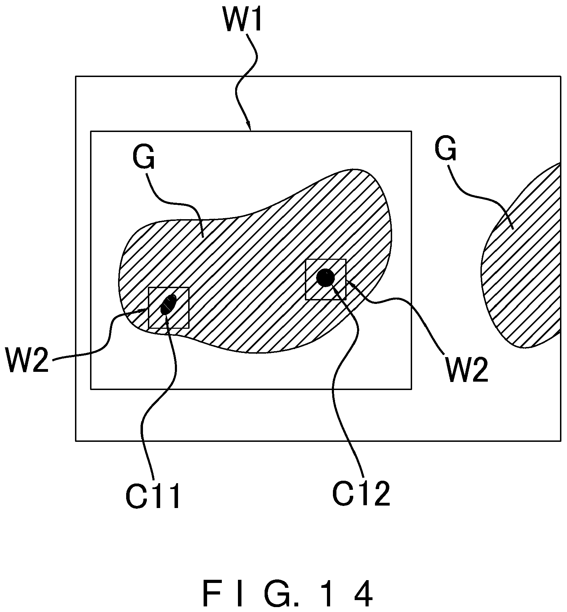

In the embodiment, as shown in FIG. 9, the regions occupied by the contaminants C11, C12 in the good G are surrounded by frames in the contaminant position identifying image of the good G. However, in a case where the good G includes the contaminants C11, C12, the entire good G may also be surrounded by a frame in the contaminant position identifying image of the good G. FIG. 14 is an example of a contaminant position identifying image of a good G in this example modification. In FIG. 14, two goods G are shown. One good G (the good G being displayed in its entirety) includes the contaminants C11, C12, and the other good G (the good G being displayed only partially) does not include contaminants. In FIG. 14, a frame W1 surrounding the entire good G that includes the contaminants C11, C12 and frames W2 surrounding each of the contaminants C11, C12 are displayed. A frame surrounding the entire good G that does not include the contaminants C11, C12 is not displayed. Because of this, workers can easily discriminate between goods that include contaminants and goods that do not include contaminants by looking at the output screens of the X-ray transmission images of the goods. As a result, the efficiency of the work of removing the contaminants from the goods G by the workers is improved, and the precision of contaminant removal is also improved.

In FIG. 14, the frame W1 and the frames W2 may also be displayed in mutually different modes. For example, the frame W1 and the frames W2 may be displayed in mutually different colors, and may be drawn with mutually different line types and thicknesses.

### (7-7) Example Modification G

The inspection device of the embodiment is the X-ray inspection device 10 that uses X-rays to perform contamination inspections of the goods G. However, the inspection device is not limited to the X-ray inspection device 10. For example, the inspection device may also be a device that uses infrared radiation, ultraviolet radiation, and visible light, for example, to perform contamination inspections of the goods G so long as it is a contaminant inspection device utilizing radiation typically used in the food production process.

### (7-8) Example Modification H

The X-ray inspection device 10 may also automatically create statistical data relating to contaminants in the goods G. The statistical data relating to contaminants are, for example, data relating to the types of the goods G and the types, positions, and shapes of contaminants.

For example, in a case where the goods G are chicken meat, the cuts (breasts, tenderloins, thighs, etc.) of chicken meat may be input before the contamination inspections are started, and statistical data about the numbers of contaminants that have been detected per cut may be created. Particularly with breast meat, often the positions where bones remain are limited to a predetermined range. For that reason, by creating statistical data per cut and using the statistical data to adjust the raw chicken cut-up device, the number of bones remaining in the chicken meat sent to the X-ray inspection device 10 can be reduced.

### (7-9) Example Modification I

In the X-ray inspection device 10, as shown in FIG. 2, regarding the goods Ga, Gb that have been discharged from the inspection line 100 and sent to the work lines 101, 102, contaminants included in the goods Ga, Gb are checked by the workers Pa, Pb, and the contaminants are removed as needed. In this case, the goods Ga, Gb from which the contaminants have been removed may be stocked and a contamination inspection may be collectively performed on them.

For example, in a case where the goods G are chicken meat, the goods G on which the contaminant removal work has been completed by each worker Pa, Pb on the work lines 101, 102 are stocked, and thereafter a contamination inspection of the goods G is collectively performed. In this case, beforehand, the workers Pa, Pb may input their own worker IDs to the X-ray inspection device 10 from the first display component 111 and the second display component 112, or the X-ray inspection device 10 may be made to read stainless steel plates and IC chips including the worker IDs. Because of this, the X-ray inspection device 10 can acquire the worker IDs of the workers Pa, Pb doing the contaminant removal work on the work lines 101, 102, so residual bone incidence rates per worker Pa, Pb can be recorded and utilized to train the workers Pa, Pb. The residual bone incidence rate is the rate at which contaminants that should be removed are mixed in with the goods G on which the contaminant removal work has been completed.

### (7-10) Example Modification J

In the embodiment and each of the above example modifications, the defects that the goods G have are classified into two types, "first defects" that are contaminants that should be removed by workers and "second defects" that do not need to be removed by workers. However, the defects that the goods G have may also be classified into three or more types.

For example, the defects that the goods G have may be classified into three types, first defects, second defects, and third defects. In a case where the defects are contaminants included in the goods G, for example, the first defects are contaminants that should be removed by workers, the second defects are contaminants that are preferred to be removed by workers, and the third defects are contaminants that do not need to be removed by workers. In this case, the defect judging component 51c discriminates which of the first to third defects the contaminants included in the goods G are, and the image adjusting component 51d adjusts the X-ray transmission images of the goods G so that the modes of display of the first to third defects mutually differ in the X-ray transmission images of the goods G.

### [Industrial Applicability]

The inspection device pertaining to the invention is applicable, for example, as an X-ray inspection device that irradiates goods such as foods with radiation such as X-rays to perform inspections of the goods.

### [Description of Reference Signs]

- 10: X-ray Inspection Device (Inspection Device)
- 51c: Defect Judging Component (Judging Component)
- 51d: Image Adjusting Component
- 51e: Threshold Setting Component (Setting Component)
- 70: Sorting Mechanism (Sorting Component)
- 111: First Display Component (Display Component)
- 112: Second Display Component (Display Component)
- G: Goods
- C: Contaminants (Defects)
- C11: Contaminant (First Defect)
- C12: Contaminant (Second Defect)

## Claims

1. An inspection device that inspects, on the basis of images of goods, the goods, the inspection device comprising:
a judging component that judges, on the basis of the images, whether or not the goods have defects;
a display component that displays the images; and
an image adjusting component that adjusts the images that are displayed on the display component,
wherein
the judging component judges whether the defects are first defects with a high need to be removed from the goods or second defects other than the first defects, and
the image adjusting component adjusts the images by configuring modes of display of the defects in the images to differ between a case where the defects include the first defects and a case where the defects do not include the first defects.

2. The inspection device of claim 1, wherein the judging component judges that the goods have defects in a case where the judging component has detected at least one of contaminants included in the goods, abnormalities in the shape of the goods, and abnormalities in the number of the goods.

3. The inspection device of claim 1 or 2, wherein the image adjusting component uses at least one of character strings, numerical values, figures, and frames surrounding predetermined regions of the images to adjust the images so that the mode of display of the first defects differs from the mode of display of the second defects.

4. The inspection device of any one of claims 1 to 3, wherein the image adjusting component adjusts the images by configuring the modes of display of the defects in the images so that entireties of the goods are surrounded by frames in a case where the defects include the first defects.

5. The inspection device of any one of claims 1 to 4, wherein the judging component judges that the goods have the first defects in a case where the goods have defects whose detection degree is higher than a predetermined threshold.

6. The inspection device of claim 5, further comprising a setting component that sets the threshold.

7. The inspection device of claim 5 or 6, wherein the image adjusting component adjusts the images so that information relating to the detection degree is included in the images.

8. The inspection device of any one of claims 5 to 7, wherein the image adjusting component adjusts the images so that the mode of display of the first defects differs from the mode of display of the second defects in accordance with the difference between the detection degree and the threshold.

9. The inspection device of any one of claims 1 to 8, wherein the image adjusting component adjusts the images so that, together with thumbnail images of the goods, enlarged images of the goods in the neighborhood of positions where there are defects are displayed on the display component.

10. The inspection device of any one of claims 1 to 9, further comprising a sorting component that sorts to a predetermined region the goods that have been judged as having defects by the judging component.
